# EUROPÄISCHE PATENTSCHRIFT

(11) **EP 4 222 146 B1**
(45) Veröffentlichungstag und Bekanntmachung des Hinweises auf die Patenterteilung: **10.07.2024**
(21) Anmeldenummer: 21798277.6
(22) Anmeldetag: 29.09.2021
(51) Int. Cl.: C07D 279/20, C08K 5/46, C09B 21/00, C09K 15/30, C10M 135/36, B60C 1/00, C10L 1/24

(54) **PHENOTHIAZIN-VERBINDUNG, IHRE HERSTELLUNG UND VERWENDUNG IN KAUTSCHUKMISCHUNGEN UND FAHRZEUGREIFEN, ALS ALTERUNGSSCHUTZMITTEL, ANTIOXIDATIONSMITTEL, ANTIOZONANT UND FARBSTOFF**
PHENOTHIAZINE COMPOUND, ITS PREPARATION AND USE IN RUBBER BLENDS AND VEHICLE TIRES, AS AGEING PROTECTANT, ANTIOXIDANT, ANTIOZONANT AND COLORANT
COMPOSÉ DE PHÉNOTHIAZINE, PRÉPARATION ET UTILISATION DE CELUI-CI DANS DES MÉLANGES DE CAOUTCHOUC ET DES PNEUMATIQUES DE VÉHICULE COMME AGENT ANTI-VIEILLISSEMENT, ANTI-OXYDANT, ANTI-OZONANT ET COLORANT

(30) Priorität: 02.10.2020 DE 102020212508
(43) Veröffentlichungstag der Anmeldung: 09.08.2023
(73) Patentinhaber: Continental Reifen Deutschland GmbH, 30175 Hannover (DE)
(72) Erfinder: RECKER, Carla, 30165 Hannover (DE); JACOB, Andreas, 30165 Hannover (DE); DAUER, David-Raphael, 3016 Hannover (DE); STROHMEIER, Julian, 30165 Hannover (DE); DREIER, Anna-Lena, 30165 Hannover (DE); BECKER, Jörg-August, 30167 Hannover (DE); MATZ, Florian, 30167 Hannover (DE); GRAF, Rebecca, 30167 Hannover (DE); FLORMANN, Jan, 30167 Hannover (DE)
(74) Vertreter: Continental Corporation
(86) Internationale Anmeldenummer: PCT/DE2021/200131
(87) Internationale Veröffentlichungsnummer: WO 2022/069001

(56) Entgegenhaltungen:
- WO-A2-2007/110627
- WO-A2-2013/132290
- US-A- 3 413 291
- US-A1- 2012 157 714
- ANDREANI, F. ET AL.: "LADDER OLIGOPHENOTHIAZINES BY DIRECT THIONATION OF N-ARYLANILINO DERIVATIVES", JOURNAL OF HETEROCYCLIC CHEMISTRY, vol. 28, 1991, pages 295 - 299, XP002516206, ISSN: 0022-152X, DOI: 10.1002/JHET.5570280215

## Beschreibung

Die Erfindung betrifft eine Verbindung, eine Kautschukmischung enthaltend die Verbindung, einen Fahrzeugreifen, der die Kautschukmischung in wenigstens einem Bauteil aufweist, Verfahren zur Herstellung der Verbindung sowie die Verwendung der Verbindung als Alterungsschutzmittel und/oder Antioxidationsmittel und/oder Antiozonant und/oder Farbstoff.

Es ist bekannt, dass in Fahrzeugreifen und technischen Gummiartikeln polymere Materialien, wie insbesondere Kautschuke, eingesetzt werden.

Naturkautschuk, synthetische Polymere (wie IR, BR, SSBR, ESBR, etc.) aber auch natürliche sowie synthetische Öle, Fette und Schmiermittel unterliegen bei längerer Lagerung und vor allem in der Zielanwendung, die oft bei höheren Temperaturen abläuft, Oxidationsreaktionen, die sich nachteilig auf die ursprünglichen gewünschten Eigenschaften auswirken. Je nach Art des Polymers werden die Polymerketten, bis hin zu einer Verflüssigung des Materials, verkürzt oder es kommt zur nachträglichen Härtung des Werkstoffes.

Alterungsschutzmittel tragen daher maßgeblich zur Langlebigkeit von Fahrzeugreifen und anderen technischen Gummiartikeln bei.

Bekannte Alterungsschutzmittel sind aromatische Amine, wie beispielsweise 6-PPD (N-(1,3-Dimethylbutyl)-N'-phenyl-p-phenylendiamin), IPPD (N-Isopropyl-N'-phenyl-p-phenylendiamin) oder SPPD (N-(1-phenylethyl)-N'-phenyl-p-phenylendiamin).

Diese Moleküle können mit Sauerstoff oder Ozon oder gebildeten Radikalen, wie Alkyl- und Alkylperoxyradikalen, reagieren und diese somit abfangen und somit die Kautschuke etc. vor weiteren Oxidationsreaktionen schützen.

Aromatische Amine weisen allerdings den Nachteil auf, dass sie im Verdacht stehen, krebserregend und damit gesundheitsschädlich zu wirken, insbesondere da Anilin oder Derivate davon freigesetzt werden können.

Zudem muss die Löslichkeit der Alterungsschutzmittel in dem zu schützenden Medium beachtet werden. Alterungsschutzmittel wie IPPD weisen den Nachteil auf, dass sie in Kautschuken teilweise schlecht löslich sind und somit an die Oberfläche migrieren und dort einen meist farbigen Film bilden. Dieser Effekt ist unter der Bezeichnung "Blooming" bekannt; das Alterungsschutzmittel blüht aus dem jeweiligen Kautschuk aus.

Nachteilig ist hieran neben der Optik auch, dass das ausgeblühte Alterungsschutzmittel beispielsweise durch Regen abgetragen wird, wodurch sich dann erneut ein Film durch weitere migrierende Moleküle bildet und somit die Konzentration des Alterungsschutzmittels in dem zu schützenden Medium kontinuierlich abnimmt. Hierdurch ergibt sich eine schlechtere Schutzwirkung als in dem Fall ohne Ausblühen und Verlust des Alterungsschutzmittels.

Alterungsschutzmittel, die insbesondere mit Ozon reagieren und dieses abfangen, werden auch als "Ozonschutzmittel" oder "Antiozonant" bezeichnet.

Der Erfindung liegt die Aufgabe zugrunde, eine Verbindung bereitzustellen, die insbesondere als Alterungsschutzmittel in Fahrzeugreifen oder anderen technischen Gummiartikeln verwendet werden kann, um somit auf Basis des Standes der Technik eine verbesserte Schutzwirkung dieser Artikel zu erzielen. Insbesondere soll das Ausblühverhalten verbessert werden und gleichzeitig eine im Vergleich zum Stand der Technik (aromatische Amine) eine weniger gesundheitsschädliche Verbindung bereitgestellt werden.

Gelöst wird die Aufgabe durch die erfindungsgemäße Verbindung gemäß Anspruch 1, die erfindungsgemäße Kautschukmischung enthaltend die Verbindung sowie den erfindungsgemäßen Fahrzeugreifen, der die erfindungsgemäße Kautschukmischung in wenigstens einem Bauteil aufweist. Ferner wird die Aufgabe durch die Verfahren zur Herstellung der Verbindung sowie die Verwendung der Verbindung als Alterungsschutzmittel und/oder Antioxidationsmittel und/oder Antiozonant gelöst.

Die Verbindung gemäß Anspruch 1 kann ferner als Farbstoff verwendet werden.

Die Verbindung gemäß Anspruch 1 weist die Formel I) auf:

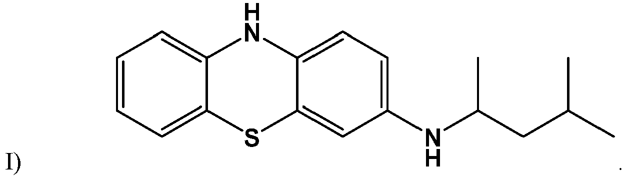

Es handelt sich bei der Verbindung gemäß Formel I) somit um 3-(1,3-Dimethylbutylamino)-phenothiazin.

Die Verbindung gemäß Formel I) ist ein Phenothiazin-Derivat. Phenothiazin bzw. Derivate davon werden unter anderem als Medikament, beispielsweise zur Bekämpfung der Parkinson-Krankheit verwendet, s. US 20130315825 A1, und sind im Vergleich zu aromatischen Aminen, wie 6PPD oder IPPD weniger gesundheitsschädlich.

Phenothiazin selbst ist allerdings nicht so gut in Polymeren, wie Kautschuken von Fahrzeugreifen oder anderen technischen Gummiartikeln, löslich, wodurch sich die beschriebene Blooming-Problematik ergibt.

Somit weist die erfindungsgemäße Verbindung gegenüber Phenothiazin den Vorteil der besseren Schutzwirkung auf.

Gleichzeitig weist die erfindungsgemäße Verbindung gemäß Formel I) gegenüber bekannten Alterungsschutzmitteln wie insbesondere 6PPD eine verbesserte Schutzwirkung auf, was mutmaßlich auf eine erhöhte Reaktivität, z. B. gegenüber Radikalen, zurückzuführen ist. Die Erfindung soll aber nicht an einen bestimmten Wirkmechanismus oder eine bestimmte Erklärung gebunden sein.

Die US 3413291 offenbart Phenothiazin-Derivate. Die darin offenbarten Derivate sind allerdings ebenfalls noch nicht gut in Kautschuken löslich, wie beispielsweise das N-isopropyl-10H-phenothiazin-3-amin.

Somit weist die erfindungsgemäße Verbindung gemäß Formel I) aufgrund der 1,3-Dimethylbutyl-Gruppe gegenüber bekannten Phenothiazin-Derivaten eine bessere Löslichkeit in Polymeren, wie Kautschuken, auf und damit eine verbesserte Schutzwirkung.

Die CN 108069874 offenbart ebenfalls Alterungsschutzmittel auf Basis von Phenothiazin-Derivaten. Allerdings stellen die darin offenbarten Derivate aufgrund der Substituenten zugleich Schiff-Basen, mit der Gruppierung R²C=NR' (R' ungleich H, Wasserstoff), dar, wie beispielsweise in Formel S1) gezeigt.

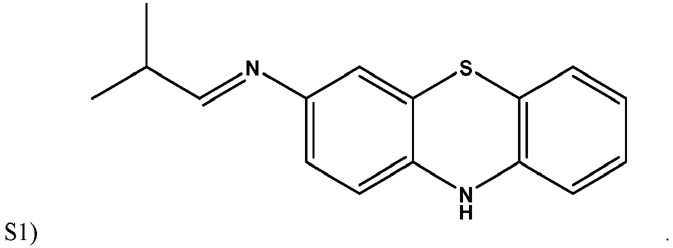

Diese Substanzen sind hydrolyseanfällig und weisen eine geringere Schutzwirkung auf, vermutlich da ein sp³-hybridisiertes α-H-Atom (alpha-Wasserstoffatom) in Nachbarschaft zur Phenyl-N-Gruppe fehlt, wie in S2) gezeigt.

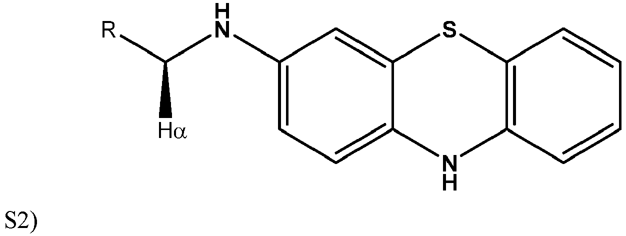

Gegenüber derartigen Schiff-Base-Phenothiazin-Derivaten, wie die Verbindung gemäß Formel S1), weist die erfindungsgemäße Verbindung gemäß Formel I) somit die Vorteile auf, dass sie stabiler, nicht hydrolyseanfällig, ist und eine bessere Schutzwirkung ermöglicht.

Die CN 106590827 offenbart ein Phenothiazin-Derivat gemäß Formel S3):

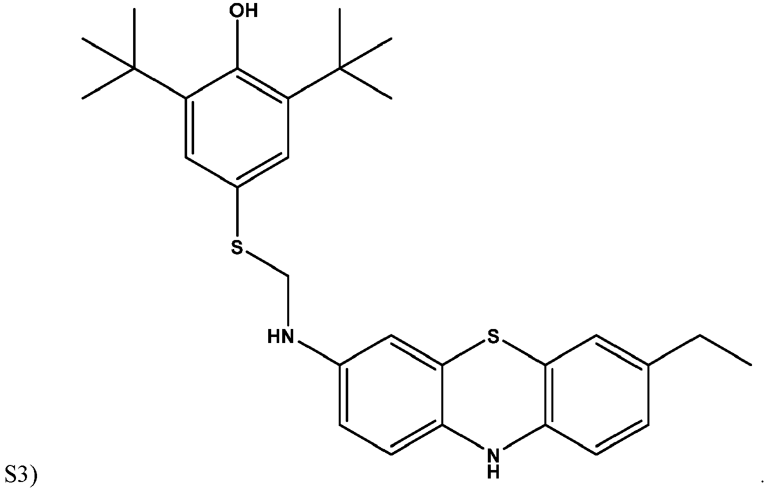

Die Verbindung gemäß Formel S3) weist allerdings den Nachteil der -S-CH₂-NH-Gruppierung auf, durch welche beispielsweise in Kautschukmischungen für Fahrzeugreifen aufgrund der Hydrolyseanfälligkeit Thiole gebildet bzw. freigesetzt werden können, welche eine unerwünschte Vorvernetzung (Scorch) der Kautschukmischung verursachen können.

Die CN 105272892 offenbart Alterungsschutzmittel wie in Formel S4) gezeigt, bei denen Phenolderivate über einen Thioacetat- sowie Amidlinker mit Phenothiazineinheiten verbunden sind. Auch diese Linkereinheiten sind für eine Verwendung in einem zu vulkanisierenden Kautschukmischungen nicht vorteilhaft, da Thioacetate verkappte Mercaptane darstellen, die bei der Vulkanisation zerfallen und beispielsweise eine Bindung zum dienhaltigen Kautschuk eingehen können. Darüber hinaus zeichnet sich die Amidgruppierung durch das Fehlen von α-H-Atomen aus, was -wie oben zu Formel S1) beschrieben - die Wirkung der Alterungsschutzmittel verringert.

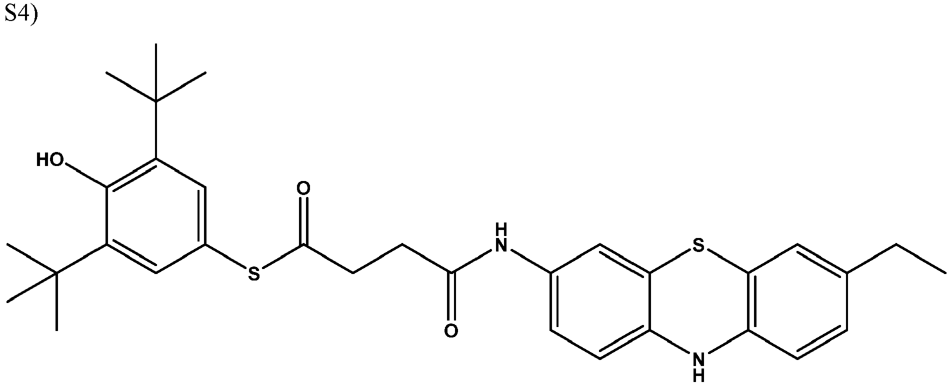

Die CN 107935867 offenbart Alterungsschutzmittel wie in Formel S5) gezeigt, welche eine freie Amin-Gruppe (-NH₂) aufweisen. Diese erhöht in einer Kautschukmischung, beispielsweise für Fahrzeugreifen oder andere technische Gummiartikel, die Gefahr der vorzeitigen Vernetzung, was auch im englischen als "scorching" bezeichnet wird.

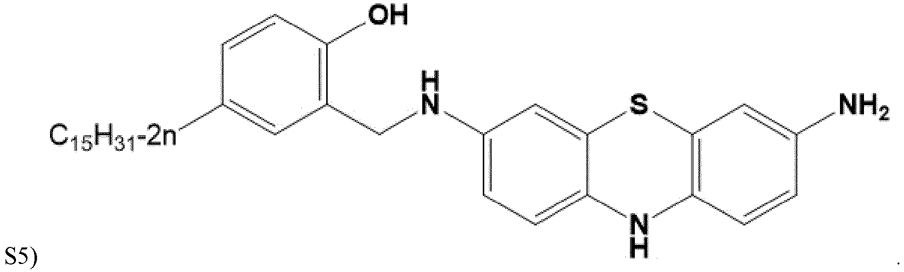

Die erfindungsgemäße Verbindung gemäß Formel I) ist besonders als Alterungsschutzmittel und/oder Ozonschutzmittel in Fahrzeugreifen und/oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen geeignet.

Die erfindungsgemäße Verbindung gemäß Formel I) ist besonders zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon geeignet.

Zu Verwendung der Verbindung gemäß Formel I) in den genannten Artikeln oder Stoffen wird diese in einer Zusammensetzung verwendet und in dieser eingemischt verwendet. Bei Fahrzeugreifen oder anderen technischen Gummiartikeln ist dies insbesondere eine Kautschukmischung.

Ein weiterer Gegenstand der Erfindung ist die Verwendung der erfindungsgemäßen Verbindung gemäß Formel I) in Ölen, Schmierstoffen, wie insbesondere Treib- oder Betriebsstoffen für Motoren. Insbesondere kann die erfindungsgemäße Verbindung somit in Motoren verwendet werden.

Ein weiterer Gegenstand der Erfindung ist die Verwendung der erfindungsgemäßen Verbindung gemäß Formel I) als Farbstoff in Fasern und/oder Polymeren und/oder Papier und/oder in (Streich-)Farben und Lacken.

Ein weiterer Gegenstand der Erfindung ist somit wie bereits aufgeführt eine Kautschukmischung.

Die erfindungsgemäße Kautschukmischung enthält die Verbindung gemäß Formel I). Bei der erfindungsgemäßen Kautschukmischung kann es sich prinzipiell um jede Kautschukmischung handeln, in der die neuartige erfindungsgemäße Verbindung gemäß Formel I) verbesserte Eigenschaften, insbesondere eine erhöhte Langlebigkeit durch Alterungsschutz und/oder Ozonschutz erzielt.

Die erfindungsgemäße Kautschukmischung enthält wenigstens einen Kautschuk.

Bevorzugt enthält die erfindungsgemäße Kautschukmischung 0,1 bis 10 phr, besonders bevorzugt 0,1 bis 5 phr, ganz besonders bevorzugt 1 bis 5 phr, der Verbindung gemäß Formel I).

Die in dieser Schrift verwendete Angabe phr (parts per hundred parts of rubber by weight) ist die in der Kautschukindustrie übliche Mengenangabe für Mischungsrezepturen. Die Dosierung der Gewichtsteile der einzelnen Substanzen wird in dieser Schrift auf 100 Gewichtsteile der gesamten Masse aller in der Mischung vorhandenen hochmolekularen (Mw größer als 20.000 g/mol) und dadurch festen Kautschuke bezogen.

Gemäß vorteilhafter Ausführungsformen der Erfindung enthält die erfindungsgemäße Kautschukmischung wenigstens einen Dienkautschuk.

Die Kautschukmischung kann somit einen Dienkautschuk oder ein Gemisch von zwei oder mehreren verschiedenen Dienkautschuken enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Der Dienkautschuk ist dabei bevorzugt ausgewählt aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Halobutyl-Kautschuk, Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, Silikon-Kautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Insbesondere Nitrilkautschuk, hydrierter Acrylnitrilbutadienkautschuk, Chloroprenkautschuk, Butylkautschuk, Halobutylkautschuk und/oder Ethylen-Propylen-Dien-Kautschuk kommen bei der Herstellung von technischen Gummiartikeln, wie Gurte, Riemen und Schläuche, und/oder Schuhsohlen zum Einsatz. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Bei dem natürlichen und/oder synthetischen Polyisopren sämtlicher Ausführungsformen kann es sich sowohl um cis-1,4-Polyisopren als auch um 3,4-Polyisopren handeln. Bevorzugt ist allerdings die Verwendung von cis-1,4-Polyisoprenen mit einem cis-1,4 Anteil > 90 Gew.-%. Zum einen kann solch ein Polyisopren durch stereospezifische Polymerisation in Lösung mit Ziegler-Natta-Katalysatoren oder unter Verwendung von fein verteilten Lithiumalkylen erhalten werden. Zum anderen handelt es sich bei Naturkautschuk (NR) um ein solches cis-1,4 Polyisopren, bei welchem der cis-1,4-Anteil im Naturkautschuk größer 99 Gew.-% ist.

Ferner ist auch ein Gemisch eines oder mehrerer natürlicher Polyisoprene mit einem oder mehreren synthetischen Polyisopren(en) denkbar.

Im Rahmen der vorliegenden Erfindung ist unter dem Begriff "Naturkautschuk" natürlich vorkommender Kautschuk zu verstehen, der von Hevea Gummibäumen und "Nicht-Hevea" Quellen gewonnen werden kann. Nicht-Hevea Quellen sind beispielsweise Guayule Sträucher und Löwenzahn wie beispielsweise TKS (Taraxacum kok-saghyz; Russischer Löwenzahn).

Falls in der erfindungsgemäßen Kautschukmischung Butadien-Kautschuk (= BR, Polybutadien) enthalten ist, kann es sich um alle dem Fachmann bekannten Typen handeln. Darunter fallen u.a. die sogenannten high-cis- und low-cis-Typen, wobei Polybutadien mit einem cis-Anteil größer oder gleich 90 Gew.-% als high-cis-Typ und Polybutadien mit einem cis-Anteil kleiner als 90 Gew.-% als low-cis-Typ bezeichnet wird. Ein low-cis-Polybutadien ist z.B. Li-BR (Lithium-katalysierter Butadien-Kautschuk) mit einem cis-Anteil von 20 bis 50 Gew.-%. Mit einem high-cis BR werden besonders gute Eigenschaften sowie eine niedrige Hysterese der Kautschukmischung erzielt.

Das oder die eingesetzte(n) Polybutadiene kann/können mit Modifizierungen und Funktionalisierungen endgruppenmodifiziert und/oder entlang der Polymerketten funktionalisiert sein. Bei der Modifizierung kann es sich um solche mit Hydroxy-Gruppen und/oder Ethoxy-Gruppen und/oder Epoxy-Gruppen und/oder Siloxan-Gruppen und/oder Amino-Gruppen und/oder Aminosiloxan und/oder Carboxy-Gruppen und/oder Phthalocyanin-Gruppen und/oder Silan-Sulfid-Gruppen handeln. Es kommen aber auch weitere, der fachkundigen Person bekannte, Modifizierungen, auch als Funktionalisierungen bezeichnet, in Frage. Bestandteil solcher Funktionalisierungen können Metallatome sein.

Für den Fall, dass wenigstens ein Styrol-Butadien-Kautschuk (Styrol-Butadien-Copolymer) in der Kautschukmischung enthalten ist, kann es sich sowohl um lösungspolymerisierten Styrol-Butadien-Kautschuk (SSBR) als auch um emulsionspolymerisierten Styrol-Butadien-Kautschuk (ESBR) handeln, wobei auch ein Gemisch aus wenigstens einem SSBR und wenigstens einem ESBR eingesetzt werden kann. Die Begriffe "Styrol-Butadien-Kautschuk" und "Styrol-Butadien-Copolymer" werden im Rahmen der vorliegenden Erfindung synonym verwendet.

Das eingesetzte Styrol-Butadien-Copolymer kann mit den oben beim Polybutadien genannten Modifizierungen und Funktionalisierungen endgruppenmodifiziert und/oder entlang der Polymerketten funktionalisiert sein.

Vorzugsweise ist der wenigstens eine Dienkautschuk ausgewählt aus der Gruppe bestehend aus natürlichem Polyisopren (NR, Naturkautschuk), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

Gemäß einer besonders bevorzugten Ausführungsform der Erfindung ist der wenigstens eine Dienkautschuk ausgewählt aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer besonders vorteilhaften Ausführungsform der Erfindung enthält die Kautschukmischung wenigstens ein natürliches Polyisopren (NR) und zwar bevorzugt in Mengen von 5 bis 55 phr, und gemäß einer besonders vorteilhaften Ausführungsform der Erfindung 5 bis 25 phr, ganz besonders bevorzugt 5 bis 20 phr. Eine derartige Kautschukmischung zeigt eine gute Verarbeitbarkeit und Reversionsstabilität sowie optimierte Reißeigenschaften und ein optimales Rollwiderstandsverhalten.

Gemäß einer besonders vorteilhaften Ausführungsform der Erfindung enthält die Kautschukmischung wenigstens ein Polybutadien (BR, Butadienkautschuk) und zwar bevorzugt in Mengen von 10 bis 80 phr, besonders bevorzugt 10 bis 50 phr, und gemäß einer besonders vorteilhaften Ausführungsform der Erfindung 15 bis 40 phr. Hiermit werden besonders gute Reiß- und Abriebeigenschaften der erfindungsgemäßen Kautschukmischung und ein optimales Bremsverhalten erzielt.

Gemäß einer besonders vorteilhaften Ausführungsform der Erfindung enthält die Kautschukmischung wenigstens einen lösungspolymerisierten Styrol-Butadien-Kautschuk (SSBR) und zwar bevorzugt in Mengen von 10 bis 80 phr, besonders bevorzugt 30 bis 80 phr, und gemäß einer besonders vorteilhaften Ausführungsform der Erfindung 50 bis 70 phr. Hiermit werden besonders gute Rollwiderstandseigenschaften der erfindungsgemäßen Kautschukmischung erzielt. Gemäß besonders vorteilhaften Ausführungsformen der Erfindung wird SSBR in Kombination mit wenigstens einem weiteren Kautschuk eingesetzt, um ein optimales und ausbalanciertes Eigenschaftsprofil zu erzielen.

Bevorzugt enthält die Kautschukmischung wenigstens einen Füllstoff, bevorzugt in Mengen von 30 bis 500 phr, besonders bevorzugt 50 bis 400 phr, wiederum bevorzugt 80 bis 300 phr.

Gemäß vorteilhafter Ausführungsformen der Erfindung ist der Füllstoff ein verstärkender Füllstoff, der bevorzugt ausgewählt ist aus der Gruppe bestehend aus Rußen und Kieselsäuren.

Gemäß besonders vorteilhafter Ausführungsformen der Erfindung enthält die Kautschukmischung wenigstens eine Kieselsäure als Füllstoff, bevorzugt in Mengen von 30 bis 500 phr, besonders bevorzugt 50 bis 400 phr, wiederum bevorzugt 80 bis 300 phr. In diesen Mengen ist Kieselsäure insbesondere als alleiniger oder als Hauptfüllstoff (mehr als 50 Gew.-% bezogen auf die Gesamtfüllstoffmenge) enthalten.

Gemäß weiterer vorteilhafter Ausführungsformen der Erfindung enthält die Kautschukmischung wenigstens eine Kieselsäure als weiteren Füllstoff, und zwar bevorzugt in Mengen von 5 bis 100 phr, besonders bevorzugt 5 bis 80 phr, wiederum bevorzugt 10 bis 60 phr.

In diesen Mengen ist Kieselsäure insbesondere als weiterer Füllstoff zusätzlich zu einem anderen Hauptfüllstoff, wie insbesondere einem Ruß, enthalten.

Bei der Kieselsäure kann es sich um die dem Fachmann bekannten Kieselsäuretypen, die als Füllstoff für Reifenkautschukmischungen geeignet sind, handeln. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 400 m²/g, bevorzugt von 35 bis 350 m²/g, besonders bevorzugt von 85 bis 320 m²/g und ganz besonders bevorzugt von 120 bis 235 m²/g, und eine CTAB-Oberfläche (gemäß ASTM D 3765) von 30 bis 400 m²/g, bevorzugt von 30 bis 330 m²/g, besonders bevorzugt von 80 bis 300 m²/g und ganz besonders bevorzugt von 115 bis 200 m²/g, aufweist. Derartige Kieselsäuren führen z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Als Kieselsäuren können somit z. B. sowohl jene des Typs Ultrasil^{®} VN3 (Handelsname) der Firma Evonik als auch hoch dispergierbare Kieselsäuren, so genannte HD-Kieselsäuren (z. B. Zeosil^{®} 1165 MP der Firma Solvay), zum Einsatz kommen.

Für den Fall, dass wenigstens zwei verschiedene Kieselsäuren, die sich z. B. durch ihre BET-Oberfläche unterscheiden, in der erfindungsgemäßem Kautschukmischung enthalten sind, beziehen sich die genannten Mengenangaben auf die Gesamtmenge aller enthaltenen Kieselsäuren.

Die Begriffe "Kieselsäure" und "Silika" werden im Rahmen der vorliegenden Erfindung synonym verwendet.

Die Kautschukmischung kann zudem weitere Füllstoffe enthalten, wie insbesondere Ruße, insbesondere Industrieruße oder Pyrolyse-Ruße, oder weitere Füllstoffe, die verstärkend wirken oder nicht verstärkend wirken.

Zu den weiteren (nicht verstärkenden) Füllstoffen zählen im Rahmen der vorliegenden Erfindung Alumosilicate, Kaolin, Kreide, Stärke, Magnesiumoxid, Titandioxid oder Kautschukgele sowie Fasern (wie zum Beispiel Aramidfasern, Glasfasern, Carbonfasern, Cellulosefasern).

Weitere ggf. verstärkende Füllstoffe sind z.B. Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und Carbonyl-Gruppen), Graphit und Graphene und sogenannte "carbon-silica dual-phase filier".

Zinkoxid gehört im Rahmen der vorliegenden Erfindung nicht zu den Füllstoffen.

Gemäß besonders vorteilhafter Ausführungsformen der Erfindung enthält die erfindungsgemäße Kautschukmischung 0,1 bis 60 phr, bevorzugt 3 bis 40 phr, besonders bevorzugt 5 bis 30 phr, ganz besonders bevorzugt 5 bis 15 phr, wenigstens eines Rußes. In diesen Mengen ist Ruß insbesondere als weiterer Füllstoff zusätzlich zu einem Hauptfüllstoff, wie insbesondere Kieselsäure, enthalten.

Gemäß weiterer vorteilhafter Ausführungsformen der Erfindung enthält die erfindungsgemäße Kautschukmischung 30 bis 300 phr, bevorzugt 30 bis 200 phr, besonders bevorzugt 40 bis 100 phr wenigstens eines Rußes. In diesen Mengen ist Ruß als alleiniger oder als Hauptfüllstoff und dabei ggf. in Kombination mit Kieselsäure in den oben genannten geringeren Mengen enthalten.

Als Ruße kommen alle der fachkundigen Person bekannten Rußtypen in Frage.

In einer Ausführungsform hat der Ruß eine Jodzahl, gemäß ASTM D 1510, die auch als Jodadsorptionszahl bezeichnet wird, zwischen 30 und 250 g/kg, bevorzugt 30 bis 180 g/kg, besonders bevorzugt 40 bis 180 g/kg, und ganz besonders bevorzugt 80 bis 150 g/kg, und eine DBP-Zahl gemäß ASTM D 2414 von 30 bis 200 ml/100 g, bevorzugt 70 bis 200 ml/100g, besonders bevorzugt 90 bis 200 ml/100g.

Die DBP-Zahl gemäß ASTM D 2414 bestimmt das spezifische Absorptionsvolumen eines Rußes oder eines hellen Füllstoffes mittels Dibutylphthalat.

Die Verwendung eines solchen Rußtyps in der Kautschukmischung, insbesondere für Fahrzeugreifen, gewährleistet einen bestmöglichen Kompromiss aus Abriebwiderstand und Wärmeaufbau, der wiederum den ökologisch relevanten Rollwiderstand beeinflusst. Bevorzugt ist hierbei, wenn lediglich ein Rußtyp in der jeweiligen Kautschukmischung verwendet wird, es können aber auch verschiedene Rußtypen in die Kautschukmischung eingemischt werden.

Gemäß einer besonders vorteilhaften Ausführungsform der Erfindung enthält die Kautschukmischung 5 bis 60 phr, besonders bevorzugt 5 bis 40 phr, wenigstens eines Rußes und 50 bis 300 phr, bevorzugt 80 bis 200 phr wenigstens einer Kieselsäure.

Des Weiteren kann die Kautschukmischung übliche Zusatzstoffe in üblichen Gewichtsteilen enthalten, die bei deren Herstellung bevorzugt in wenigstens einer Grundmischstufe zugegeben werden. Zu diesen Zusatzstoffen zählen
a) im Stand der Technik bekannte Alterungsschutzmittel, wie z. B. Diamine, wie N-Phenyl-N'-(1,3-dimethylbutyl)-p-phenylendiamin (6PPD), N,N`-Diphenyl-p-phenylendiamin (DPPD), N,N'-Ditolyl-p-phenylendiamin (DTPD), N-(1,4-dimethylpentyl)-N'-phenyl-p-phenylendiamin (7PPD), N-Isopropyl-N'-phenyl-p-phenylendiamin (IPPD), oder Dihydrochinoline, wie 2,2,4-Trimethyl-1,2-dihydrochinolin (TMQ),
b) Aktivatoren, wie z. B. Zinkoxid und Fettsäuren (z. B. Stearinsäure) und/oder sonstige Aktivatoren, wie Zinkkomplexe wie z.B. Zinkethylhexanoat,
c) Aktivatoren und/oder Agenzien für die Anbindung von Füllstoffen, insbesondere Ruß oder Kieselsäure, wie beispielsweise S-(3-Aminopropyl)Thioschwefelsäure und/oder deren Metallsalze (Anbindung an Ruß) sowie Silan-Kupplungsagenzien (Anbindung an Kieselsäure),
d) Ozonschutzwachse,
e) Harze, insbesondere Klebharze für innere Reifenbauteile,
f) Mastikationshilfsmittel, wie z. B. 2,2'-Dibenzamidodiphenyldisulfid (DBD) und
g) Prozesshilfsmittel, wie insbesondere Fettsäureester und Metallseifen, wie z.B. Zinkseifen und/oder Calciumseifen
h) Weichmacher, wie insbesondere aromatische, naphthenische oder paraffinische Mineralölweichmacher, wie z.B. MES (mild extraction solvate) oder RAE (Residual Aromatic Extract) oder TDAE (treated distillate aromatic extract), oder Rubber-to-Liquid-Öle (RTL) oder Biomass-to-Liquid-Öle (BTL) bevorzugt mit einem Gehalt an polycyclischen Aromaten von weniger als 3 Gew.-% gemäß Methode IP 346 oder Triglyceride, wie z. B. Rapsöl, oder Faktisse oder Kohlenwasserstoffharze oder Flüssig-Polymere, deren mittleres Molekulargewicht (Bestimmung per GPC = gel permeation chromatography, in Anlehnung an BS ISO 11344:2004) zwischen 500 und 20000 g/mol liegt.

Bei der Verwendung von Mineralöl ist dieses bevorzugt ausgewählt aus der Gruppe, bestehend aus DAE (Destillated Aromatic Extracts) und/oder RAE (Residual Aromatic Extract) und/oder TDAE (Treated Destillated Aromatic Extracts) und/oder MES (Mild Extracted Solvents) und/oder naphthenische Öle.

Gemäß besonders vorteilhafter Ausführungsformen enthält die erfindungsgemäße Kautschukmischung neben den erfindungsgemäßen gemäß Formel I) keine weiteren Alterungsschutzmittel aus der Gruppe der p-Phenylendiamine, s. obige Auflistung a). Insbesondere enthält die erfindungsgemäße Kautschukmischung gemäß einer besonders bevorzugten Ausführungsform 0 bis 0,1 phr, insbesondere 0 phr, an weiteren Alterungsschutzmitteln auf Basis von Diaminen, die ausgewählt sind aus der Gruppe enthaltend, bevorzugt bestehend aus, N-Phenyl-N'-(1,3-dimethylbutyl)-p-phenylendiamin (6PPD), N,N`-Diphenyl-p-phenylendiamin (DPPD), N,N'-Ditolyl-p-phenylendiamin (DTPD), N-Isopropyl-N'-phenyl-p-phenylendiamin (IPPD), N-(1,4-dimethylpentyl)-N'-phenyl-p-phenylendiamin (7PPD).

Mit den bevorzugt sehr geringen Mengen von 0 bis 0,1 phr bzw. besonders bevorzugt 0 phr an den genannten Diaminen und der erfindungsgemäß enthaltenen Verbindung gemäß Formel I) ist es möglich eine verbesserte Schutzwirkung zu erzielen. Hierbei ersetzt die erfindungsgemäße Verbindung gemäß Formel I) die genannten im Stand der Technik bekannten Diamine.

Gemäß weiterer vorteilhafter Ausführungsformen der Erfindung ist noch wenigstens ein weiteres der genannten Diamin-Alterungsschutzmittel enthalten, sodass die erfindungsgemäße Verbindung die im Stand der Technik bekannten Diamine nur teilweise ersetzt. Hierdurch wird der erfindungsgemäße Vorteil auch erzielt, nur nicht in optimalem Ausmaß.

Alterungsschutzmittel auf Basis von Dihydrochinolin, wie TMQ, sind gemäß vorteilhafter Ausführungsformen neben der erfindungsgemäßen Verbindung gemäß Formel I) in der Kautschukmischung enthalten. Die Menge an enthaltenen Dihydrochinolinen, wie insbesondere TMQ, beträgt bevorzugt 0,1 bis 3, insbesondere 0,5 bis 1,5 phr.

Gemäß weiterer vorteilhafter Ausführungsformen enthält die erfindungsgemäße Kautschukmischung keine weiteren Alterungsschutzmittel, d. h. 0 phr an weiteren Alterungsschutzmitteln außer der erfindungsgemäßen Verbindung gemäß Formel I).

Bei den Silan-Kupplungsagenzien kann es sich um alle dem Fachmann bekannten Typen handeln.

Ferner können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen der Kieselsäure oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung).

Aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln:
-SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3 `-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT in verschiedenen Varianten von der Firma Momentive, USA, oder solche, die unter dem Namen VP Si 363^{®} von der Firma Evonik Industries vertrieben werden.

Der Mengenanteil der Gesamtmenge an weiteren Zusatzstoffen beträgt bevorzugt 3 bis 150 phr, besonders bevorzugt 3 bis 100 phr und ganz besonders bevorzugt 5 bis 80 phr.

Im Gesamtmengenanteil der weiteren Zusatzstoffe kann Zinkoxid (ZnO) in den oben genannten Mengen enthalten sein.

Hierbei kann es sich um alle dem Fachmann bekannten Typen an Zinkoxid handeln, wie z.B. ZnO-Granulat oder -Pulver. Das herkömmlicherweise verwendete Zinkoxid weist in der Regel eine BET-Oberfläche von weniger als 10 m²/g auf. Es kann aber auch ein Zinkoxid mit einer BET-Oberfläche von 10 bis 100 m²/g, wie z.B. so genannte "nano-Zinkoxide", verwendet werden.

Die erfindungsgemäße Kautschukmischung wird bevorzugt vulkanisiert verwendet, insbesondere in Fahrzeugreifen oder anderen vulkanisierten technischen Gummiartikeln.

Die Vulkanisation der erfindungsgemäßen Kautschukmischung wird bevorzugt in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern und/oder Mercaptobeschleunigern und/oder Sulfenamidbeschleunigern und/oder Thiocarbamatbeschleunigern und/oder Thiurambeschleunigern und/oder Thiophosphatbeschleunigern und/oder Thioharnstoffbeschleunigern und/oder Xanthogenat-Beschleunigern und/oder Guanidin-Beschleunigern.

Bevorzugt ist die Verwendung eines Sulfenamidbeschleunigers, der ausgewählt ist aus der Gruppe bestehend aus N-Cyclohexyl-2-benzothiazolsufenamid (CBS) und/oder N,N-Dicyclohexylbenzothiazol-2-sulfenamid (DCBS) und/oder Benzothiazyl-2-sulfenmorpholid (MBS) und/oder N-tert-Butyl-2-benzothiazylsulfenamid (TBBS) oder eines Guanidin-Beschleunigers wie Diphenylguanidin (DPG).

Als schwefelspendende Substanz können dabei alle dem Fachmann bekannten schwefelspendenden Substanzen verwendet werden. Enthält die Kautschukmischung eine schwefelspendende Substanz, ist diese bevorzugt ausgewählt aus der Gruppe enthaltend z.B. Thiuramdisulfide, wie z.B. Tetrabenzylthiuramdisulfid (TBzTD) und/oder Tetramethylthiuramdisulfid (TMTD) und/oder Tetraethylthiuramdisulfid (TETD), und/oder Thiuramtetrasulfide, wie z.B. Dipentamethylenthiuramtetrasulfid (DPTT), und/oder Dithiophosphate, wie z.B.

DipDis (Bis-(Diisopropyl)thiophosphoryldisulfid) und/oder Bis(O,O-2-ethylhexyl-thiophosphoryl)Polysulfid (z. B. Rhenocure SDT 50^{®}, Rheinchemie GmbH) und/oder Zinkdichloryldithiophosphat (z. B. Rhenocure ZDT/S^{®}, Rheinchemie GmbH) und/oder Zinkalkyldithiophosphat, und/oder 1,6-Bis(N,N-dibenzylthiocarbamoyldithio)hexan und/oder Diarylpolysulfide und/oder Dialkylpolysulfide.

Auch weitere netzwerkbildende Systeme, wie sie beispielsweise unter den Handelsnamen Vulkuren^{®}, Duralink^{®} oder Perkalink^{®} erhältlich sind, oder netzwerkbildende Systeme, wie sie in der WO 2010/049216 A2 beschrieben sind, können in der Kautschukmischung eingesetzt werden. Dieses System enthält ein Vulkanisationsmittel, welches mit einer Funktionalität größer vier vernetzt und zumindest einen Vulkanisationsbeschleuniger.

Besonders bevorzugt ist die Verwendung der Beschleuniger TBBS und/oder CBS und/oder Diphenylguanidin (DPG).

Außerdem können in der Kautschukmischung Vulkanisationsverzögerer vorhanden sein.

Die Begriffe "vulkanisiert" und "vernetzt" werden im Rahmen der vorliegenden Erfindung synonym verwendet.

Gemäß einer bevorzugten Weiterbildung der Erfindung werden bei der Herstellung der schwefelvernetzbaren Kautschukmischung mehrere Beschleuniger in der Fertigmischstufe zugegeben.

Die Herstellung der erfindungsgemäßen schwefelvernetzbaren Kautschukmischung erfolgt nach dem in der Kautschukindustrie üblichen Verfahren, bei dem zunächst in ein oder mehreren Mischstufen eine Grundmischung mit allen Bestandteilen außer dem Vulkanisationssystem (Schwefel und vulkanisationsbeeinflussende Substanzen) hergestellt wird. Durch Zugabe des Vulkanisationssystems in einer letzten Mischstufe wird die Fertigmischung erzeugt. Die Fertigmischung wird z.B. durch einen Extrusionsvorgang oder Kalandrieren weiterverarbeitet und in die entsprechende Form gebracht. Anschließend erfolgt die Weiterverarbeitung durch Vulkanisation, wobei aufgrund des im Rahmen der vorliegenden Erfindung zugegebenen Vulkanisationssystems eine Schwefelvernetzung stattfindet.

Die oben beschriebene erfindungsgemäße Kautschukmischung ist besonders für die Verwendung in Fahrzeugreifen, insbesondere Fahrzeugluftreifen geeignet.

Zur Verwendung in Fahrzeugreifen wird die Mischung als Fertigmischung vor der Vulkanisation bevorzugt in die Form eines Laufstreifens gebracht und bei der Herstellung des Fahrzeugreifenrohlings wie bekannt aufgebracht.

Die Herstellung der erfindungsgemäßen Kautschukmischung zur Verwendung als Seitenwand oder sonstige Body- Mischung in Fahrzeugreifen erfolgt wie bereits beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Als Body-Mischung werden hierbei die Kautschukmischungen für die inneren Bauteile eines Reifen bezeichnet, wie im Wesentlichen Squeegee, Innenseele (Innenschicht), Kernprofil, Gürtel, Schulter, Gürtelprofil, Karkasse, Wulstverstärker, Wulstprofil, Hornprofil und Bandage. Der noch unvulkanisierte Reifenrohling wird anschließend vulkanisiert.

Zur Verwendung der erfindungsgemäßen Kautschukmischung in Riemen und Gurten, insbesondere in Fördergurten, wird die extrudierte noch unvulkanisierte Mischung in die entsprechende Form gebracht und dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

Ein weiterer Gegenstand der vorliegenden Erfindung ist ein Fahrzeugreifen, der die erfindungsgemäße Kautschukmischung enthaltend die erfindungsgemäße Verbindung in wenigstens einem Bauteil aufweist.

Der vulkanisierte Fahrzeugreifen weist wenigstens in einem Bauteil ein Vulkanisat wenigstens einer erfindungsgemäßen Kautschukmischung auf. Dem Fachmann ist bekannt, dass die meisten Substanzen, wie z. B. die enthaltenen Kautschuke entweder bereits nach dem Mischen oder erst nach der Vulkanisation in chemisch veränderter Form vorliegen.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Bevorzugt weist der erfindungsgemäße Fahrzeugreifen die erfindungsgemäße Kautschukmischung in wenigstens einem äußeren Bauteil auf, wobei das äußere Bauteil bevorzugt ein Laufstreifen, eine Seitenwand und/oder ein Hornprofil ist.

Der erfindungsgemäße Fahrzeugreifen kann die erfindungsgemäße Kautschukmischung enthaltend die erfindungsgemäße Verbindung gemäß Formel I) somit auch in mehreren Bauteilen in ggf. angepasster Zusammensetzung aufweisen.

Ein weiterer Gegenstand der vorliegenden Erfindung ist ein Verfahren zur Herstellung der Verbindung gemäß Formel I), wobei das Verfahren wenigstens die folgenden Verfahrensschritte umfasst:
a) Bereitstellung der Substanz gemäß Formel A) 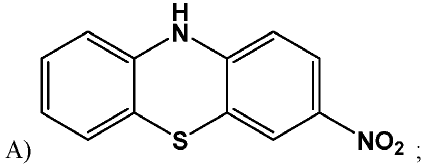
b) Bereitstellung von Methylisobutylketon (MIBK) und Wasserstoff (H₂) ;
c) Umsetzung der Substanz gemäß Schritt a) mit den Substanzen gemäß Schritt b), bevorzugt in Gegenwart eines Hydrierungskatalysators, zu der Substanz gemäß Formel I)

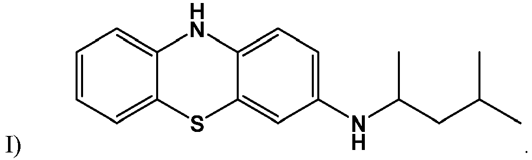

Ein weiterer Gegenstand der vorliegenden Erfindung ist ein weiteres Verfahren zur Herstellung der Verbindung gemäß Formel I), wobei das Verfahren wenigstens die folgenden Verfahrensschritte umfasst:
a1) Bereitstellung der Substanz gemäß Formel A) 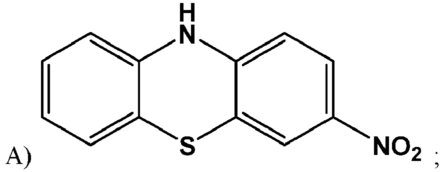
b1) Bereitstellung eines Reduktionsmittels, insbesondere Zinn(II)chlorid-Dihydrat;
c1) Umsetzung der Substanz gemäß Schritt a1) mit der Substanz aus Schritt b1) zu der Substanz gemäß Formel C1) 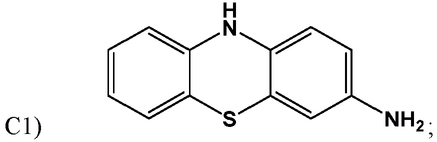
d1) Bereitstellung von Methylisobutylketon (MIBK) und Wasserstoff (H₂);
e1) Umsetzung der Substanz gemäß Formel C1) mit den Substanzen gemäß Schritt d1), bevorzugt in Gegenwart eines Hydrierungskatalysators, zu der Substanz gemäß Formel I)

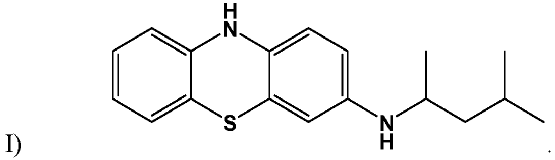

Die Substanz Methylisobutylketon (MIBK) ist kommerziell erhältlich.

Bei dem Reduktionsmittel kann es sich um jedes dem Fachmann bekannte Reduktionsmittel handeln, welches geeignet ist, die Nitro-Gruppe (-NO₂) zu einer AminoGruppe (-NH₂) zu reduzieren. Geeignet ist insbesondere Zinn(II)chlorid-Dihydrat. Dieses ist kommerziell erhältlich.

Das Verfahren gemäß der Schritte a) bis c) ist gegenüber dem Verfahren gemäß der Schritte a1) bis e1) bevorzugt, da weniger Verfahrensschritte notwendig sind.

Bevorzugt wird in den Verfahrensschritten, in denen eine Umsetzung mit Wasserstoff erfolgt, ein geeigneter Katalysator, im Rahmen der vorliegenden Erfindung als "Hydrierungskatalysator" bezeichnet, verwendet.

Bevorzugt ist der Hydrierungskatalysator der Verfahren (Schritt c) bzw. e1)) ein Edelmetallkatalysator, wie insbesondere Palladium (Pd) oder Platin (Pt). Bevorzugt wird das Edelmetall auf Kohle (C) eingesetzt, wie Palladium auf Kohle (Pd/C) oder Platin auf Kohle (Pt/C).

Ferner können auch andere bekannte Katalysatoren, wie Raney-Nickel oder Kupferchromit verwendet werden.

Bevorzugt wird in Schritt c) Pt/C verwendet.

Bevorzugt wird in Schritt e1) Pd/C verwendet.

Der Wasserstoffdruck bei den jeweiligen Verfahrensschritten, bei denen Wasserstoff verwendet wird, beträgt bevorzugt 1 bis 50 bar, besonders bevorzugt 10 bis 45 bar. Gemäß einer vorteilhaften Ausführungsform der Erfindung sind 20 bis 40 bar bevorzugt.

Die Hydrierungsreaktion in Schritt c) bzw. e1) findet bevorzugt in einem Autoklaven, insbesondere einem Edelstahlautoklaven, statt.

Die Temperatur in Verfahrensschritt c) sowie e1) beträgt bevorzugt von Raumtemperatur (RT, insbesondere 20 °C) bis 150 °C, bevorzugt bis 130 °C.

Ein weiterer Gegenstand der vorliegenden Erfindung ist ein weiteres Verfahren zur Herstellung der Verbindung gemäß Formel I), wobei das Verfahren wenigstens die folgenden Verfahrensschritte umfasst:
a2) Bereitstellung der Substanz gemäß Formel A2): 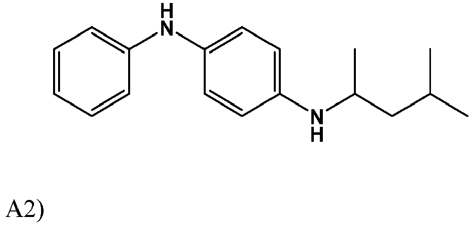
b2) Bereitstellung von elementarem Schwefel und o-Dichlorbenzol (ortho-Dichlorbenzol);
c2) Umsetzung der Substanz gemäß Schritt a2) mit den Substanzen gemäß Schritt b2) zu der Substanz gemäß Formel I)

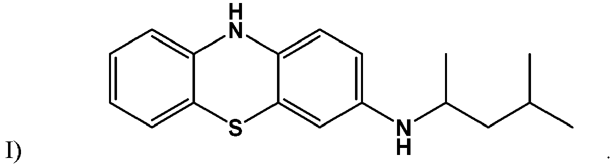

Im Folgenden soll die Erfindung anhand von Ausführungsbeispielen näher erläutert werden.

Die Verbindung gemäß Formel I) wurde auf folgende Weisen hergestellt:

### X1) Synthese der erfindungsgemäßen Verbindung gemäß Formel I) (3-(1,3-Dimethylbutylaminol-phenothiazin) gemäß den Verfahrensschritten a) bis c):

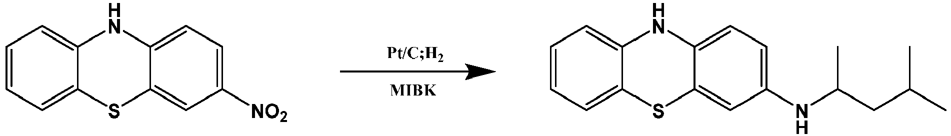

In einen Edelstahlautoklaven, der mit einem Tefloninliner bestückt wurde, wurden 0.50 g (2.05 mmol, 1 Äq) 3-Nitrophenothiazin, 0.18 g Platin auf Kohle (5%) (0.4 g auf 4.67 mmol Substrat) und 20.0 mL Methylisobutylketon eingewogen. Anschließend wurde 40 bar Wasserstoff aufgedrückt und bei 120°C für 10 Stunden rühren gelassen. Nach Beendigung der Reaktion wurde der überschüssige Wasserstoff abgeblasen und die Suspension über Kieselgur (Celite^{®}) filtriert sowie mit Ethanol nachgewaschen. Das Filtrat wurde bis zur Trockne eingeengt und im Vakuum getrocknet. Gräulicher bis violetter Feststoff; Ausbeute 0.57 g (93 % d. Th.).

¹H-NMR (*engl.* "nuclear magnetic resonance") (500 MHz, DMSO-*d6*) δ = 8.09 (s, 1H), 6.94 (td, *J* = 7.6, 1.5 Hz, 1H), 6.88 (dd, *J* = 7.6, 1.4 Hz, 1H), 6.69 - 6.63 (m, 2H), 6.51 (d, *J* = 8.4 Hz, 1H), 6.27 (dd, *J* = 8.5, 2.5 Hz, 1H), 6.21 (d, *J* = 2.5 Hz, 1H), 4.82 (d, *J* = 8.9 Hz, 1H), 3.30 (dt, *J* = 8.6, 6.5 Hz, 1H), 1. 70 (dp, *J* = 13.5, 6.7 Hz, 1H), 1.38 (dt, *J* = 13.9, 7.1 Hz, 1H), 1.16 (dt, *J* = 13.5, 6.8 Hz, 1H), 1.02 (d, *J* = 6.1 Hz, 3H), 0.87 (dd, *J* = 19.4, 6.6 Hz, 6H).

¹³C-NMR (126 MHz, DMSO-*d6*) δ = 144.5, 143.9, 131.8, 127.7, 126.6, 121.0, 117.6, 116.5, 115.8, 114.4, 112.1, 110.7, 46.5, 46.4, 25.0, 23.2, 23.0, 21.2.

ESI-MS (Elektrosprayionisation Massenspektrometrie) [M+H]⁺ = 299.

In einem weiteren Versuch X2) wurde die Reaktion wie oben beschrieben durchgeführt, nur mit den Unterschieden, dass ebenfalls bei 40 bar Wasserstoff aufgedrückt aber bei 40 °C für 3 Stunden rühren gelassen wurde.

Es ergibt sich eine ähnliche Ausbeute, wie die nachfolgende Tabelle 1 darstellt.

**Tabelle 1**

| **Nr.** | **Temperatur [°C]** | **Druck [bar]** | **Dauer [h]** | **Ausbeute [%]** |
|---|---|---|---|---|
| **X1** | 120 | 40 | 10 | 99 |
| **X2** | 40 | 40 | 3 | 97 |

Die Ausgangssubstanz 3-Nitrophenothiazin, Substanz gemäß Formel A) nach obiger Beschreibung des Verfahrens, wurde gemäß der Offenbarung in der US 20130315825 A1 hergestellt.

Alternativ kann 3-Nitrophenothiazin über eine einstufige Kupfer- (Cu) katalysierte Synthese, wie in der WO 2017011531 A2 (S. 217) offenbart, hergestellt werden.

Ferner ist es möglich 3-Nitrophenothiazin durch eine unkatalysierte Reaktion, veröffentlicht von S. Wu, W. Hu, S. Zhang, RSC Advances, 2016, 6(29), 24257-24260, zu erhalten.

Ferner kann 3-Nitrophenothiazin aus Phenothiazin, wie in der WO 2007110627 A2 offenbart, durch Umsetzung mit Natriumnitrit erhalten werden.

Alternativ wurde die Substanz gemäß Formel I) in einer zweistufigen Reaktion gemäß den Verfahrensschritten a1) bis e1) ausgehend von 3-Nitrophenothiazin erhalten:

### Synthese von 3-Aminophenothiazin:

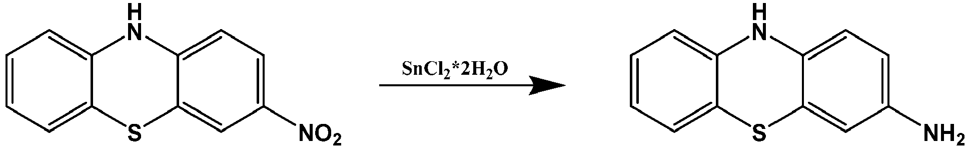

Unter Schutzgas wurden 22g (90.0 mmol, 1.0 Äq) 3-Nitro-Phenothiazin eingewogen, in 800 mL Ethanol gelöst, mit 102 g (534 mmol, 5.0 Äq) Zinn(II)chlorid-Dihydrat versetzt und für 4 Tage in der Siedehitze gerührt. Nachdem die Reaktion auf Raumtemperatur (RT) gebracht wurde, wurde ein Drittel des Lösungsmittels abdestilliert und der Rest auf Eis gegossen. Anschließend wurde der pH-Wert der Mischung mittels Kalilauge auf pH=7 eingestellt und dreimal mit Essigester extrahiert. Die organische Phase wurde mit gesättigter NaCl-Lösung (Lsg.) gewaschen und anschließend über Na₂SO₄ getrocknet. Es wurde ein brauner Feststoff erhalten; Ausbeute 18.9 g (98 % d. Th.).

¹H-NMR (500 MHz, DMSO-*d6*) δ = 8.08 (s, 1H), 6.94 (td, *J* = 7.7, 1.5 Hz, 1H), 6.88 (dd, *J* = 7.7, 1.5 Hz, 1H), 6.71 - 6.60 (m, 2H), 6.46 (d, *J* = 8.3 Hz, 1H), 6.32 - 6.22 (m, H), 4.64 (s, 2H).

ESI-MS [M+H]⁺ = 215.

### Synthese von 3-(1,3-Dimethylbulylamino)-phenothiazin:

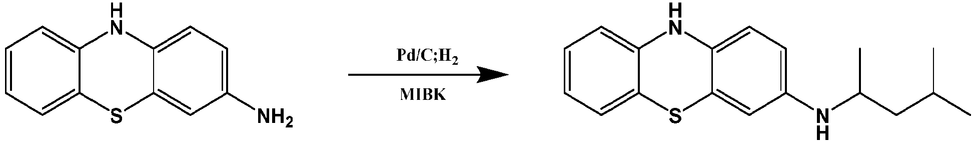

In einen Edelstahlautoklaven, der mit einem Tefloninliner bestückt wurde, wurden 5.00 g (23.3 mmol, 1 Äq) 3-Aminophenothiazin, 2.00 g Palladium auf Kohle (5%) (0.4 g auf 4.67 mmol Substrat) und 50.0 mL Methylisobutylketon eingewogen. Anschließend wurde 40 bar Wasserstoff aufgedrückt und bei 120°C für 10 Stunden rühren gelassen. Nach Beendigung der Reaktion wurde der überschüssige Wasserstoff abgeblasen und die Suspension über Kieselgur (Celite^{®}) filtriert sowie mit Ethanol nachgewaschen. Das Filtrat wurde bis zur Trockne eingeengt und im Vakuum getrocknet. Gräulicher bis violetter Feststoff; Ausbeute 6.61 g (95 % d. Th.).

¹H-NMR (500 MHz, DMSO-*d6*) δ = 8.09 (s, 1H), 6.94 (td, *J* = 7.6, 1.5 Hz, 1H), 6.88 (dd, *J* = 7.6, 1.4 Hz, 1H), 6.69 - 6.63 (m, 2H), 6.51 (d, *J* = 8.4 Hz, 1H), 6.27 (dd, *J* = 8.5, 2.5 Hz, 1H), 6.21 (d, *J* = 2.5 Hz, 1H), 4.82 (d, *J* = 8.9 Hz, 1H), 3.30 (dt, *J* = 8.6, 6.5 Hz, 1H), 1.70 (dp, *J* = 13.5, 6.7 Hz, 1H), 1.38 (dt, *J* = 13.9, 7.1 Hz, 1H), 1.16 (dt, *J* = 13.5, 6.8 Hz, 1H), 1.02 (d, *J* = 6.1 Hz, 3H), 0.87 (dd, *J* = 19.4, 6.6 Hz, 6H).

¹³C-NMR (126 MHz, DMSO-*d6*) δ = 144.5, 143.9, 131.8, 127.7, 126.6, 121.0, 117.6, 116.5, 115.8, 114.4, 112.1, 110.7, 46.5, 46.4, 25.0, 23.2, 23.0, 21.2.

ESI-MS [M+H]⁺ = 299.

Alternativ wurde die Substanz gemäß Formel I) in einer einstufigen Reaktion gemäß den Verfahrensschritten a2) bis c2) erhalten:

### Synthese von 3-(1,3-Dimethylbutylamino)-phenothiazin:

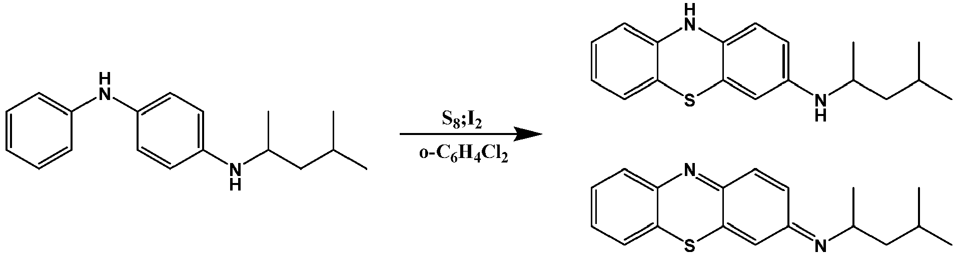

Unter Schutzgas wurden 10 g (37.3 mmol, 1 Äq) 6-PPD (N-(1,3-Dimethylbutyl)-N'-phenyl-p-phenylendiamin) in 80 mL entgastem o-Dichlorbenzol gelöst und mit 2.9 g (89 mmol, 2.4 Äq bezogen auf monomeren "S") Schwefel sowie 0.95 g (3.73 mmol, 0.1 Äq) Iod (I₂) versetzt. Die Mischung wurde für 4 Stunden unter Rückfluss (180°C) erhitzt. Entstehender Schwefelwasserstoff wurde dabei in eine 5%ige NaOH-Lsg. geleitet. Nachdem auf Raumtemperatur (RT) abgekühlt wurde, wurde die Mischung mit Dichlormethan (DCM) und einer gesättigten (ges.) Na₂S₂O₃ versetzt. Der dabei entstandene schwarze Feststoff wurde per Filtration entfernt und die organische Phase von der wässrigen separiert. Die organische Phase wurde mit ges. NaCl-Lsg. gewaschen, über Na₂SO₄ getrocknet und zur Trockne eingeengt. Der schwarze Feststoff wurde per LC-MS (Liquid-Chromatographie-Massenspektrometrie) mit gekoppelter UV-VIS-Spektroskopie (Abk. von *engl.* "ultraviolet and visible spectroscopy") analysiert. Es enthielt 39 % (E)-N-(4-methylpentan-2-yl)-3H-phenothiazin-3-imine als Nebenprodukt (unteres Molekül auf der rechten Seite des Reaktionspfeiles) sowie 24 % undefinierte Substanzen. Die restlichen 37 % setzen sich aus Startmaterial sowie der erfindungsgemäßen Verbindung gemäß Formel I) zusammen.

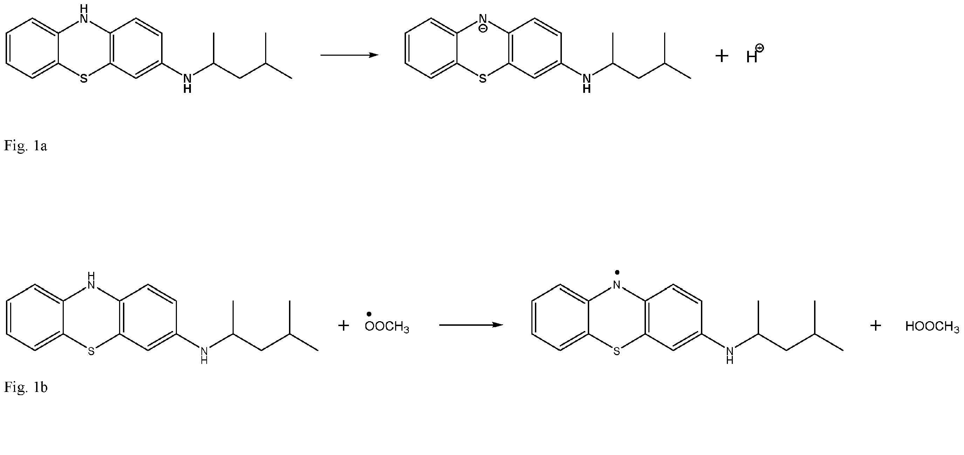

Die erfindungsgemäße Verbindung gemäß Formel I) weist ggü. 6PPD eine erhöhte Reaktivität, auf. Dies ließ sich beispielsweise anhand der Berechnung von Bindungsdissoziationsenergien (BDE), sowie der freien Aktivierungsenthalpie Δ_{R}G^{≠} bzw. der freien Standardreaktionsenthalpie Δ_{R}G° für die Reaktion mit einem Methylperoxidradikal nachvollziehen. Die Werte sind in Tabelle 2 angegeben. In Fig.1a und 1b sind die Spaltmechanismen dargestellt, auf die sich die Werte beziehen.

**Tabelle 2**

| **Molekül** | **BDE [kJ/mol]** | **Δ_{R}G° [kJ/mol]** | **Δ_{R}G^{≠} [kJ/mol]** |
|---|---|---|---|
| 6PPD | 313 | -25,6 | 19,1 |
| Formel I) | 251 | -52,8 | 6,3 |

Wie Tabelle 2 zeigt, ergeben sich für die erfindungsgemäße Verbindung gemäß Formel I) eine niedrigere Bindungsdissoziationsenergie und niedrigere freie Enthalpien.

Mit der Verbindung gemäß Formel I) kann somit eine verbesserte Schutzwirkung bei den genannten Anwendungsmöglichkeiten erzielt werden.

Für die Anwendung in einer Kautschukmischung für Fahrzeugreifen wird die erfindungsgemäße Verbindung gemäß Formel I) beispielsweise anstelle der im Stand der Technik bekannten Alterungsschutzmittel, wie 6PPD, 7PPD oder IPPD usw., auf dem Fachmann bekannte Weise in einer der Mischstufen bei der Herstellung der Kautschukmischung zugegeben.

## Patentansprüche

1. Verbindung gemäß Formel I):

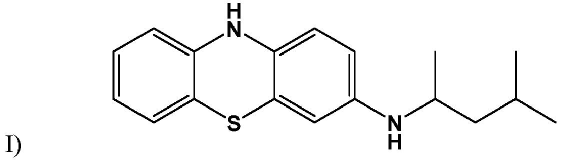

2. Kautschukmischung enthaltend die Verbindung gemäß Formel I) nach Anspruch 1.

3. Kautschukmischung nach Anspruch 2, **dadurch gekennzeichnet, dass** sie wenigstens einen Dienkautschuk enthält.

4. Kautschukmischung nach Anspruch 3, **dadurch gekennzeichnet, dass** sie wenigstens einen Dienkautschuk enthält, der ausgewählt ist aus der Gruppe bestehend aus natürlichem Polyisopren (NR), synthetischem Polyisopren (IR), Butadienkautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

5. Fahrzeugreifen, der die Kautschukmischung nach einem der Ansprüche 2 bis 4 in wenigstens einem Bauteil aufweist.

6. Fahrzeugreifen nach Anspruch 5, **dadurch gekennzeichnet, dass** er wenigstens eine Kautschukmischung nach einem der Ansprüche 2 bis 4 in wenigstens einem äußeren Bauteil aufweist, wobei das äußere Bauteil bevorzugt ein Laufstreifen, eine Seitenwand und/oder ein Hornprofil ist.

7. Verfahren zur Herstellung der Verbindung gemäß Formel I) nach Anspruch 1 umfassend wenigstens die folgenden Verfahrensschritte:
a) Bereitstellung der Substanz gemäß Formel A) 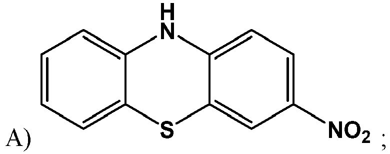
b) Bereitstellung von Methylisobutylketon (MIBK) und Wasserstoff (H₂) ;
c) Umsetzung der Substanz gemäß Schritt a) mit den Substanzen gemäß Schritt b) zu der Substanz gemäß Formel I)

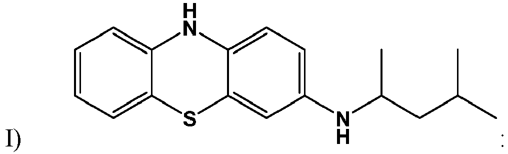

8. Verfahren zur Herstellung der Verbindung gemäß Formel I) nach Anspruch 1 umfassend wenigstens die folgenden Verfahrensschritte:
a1) Bereitstellung der Substanz gemäß Formel A) 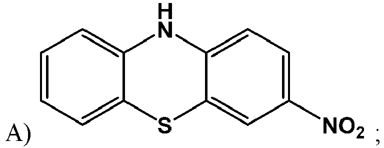
b1) Bereitstellung eines Reduktionsmittels, insbesondere Zinn(II)chlorid-Dihydrat;
c1) Umsetzung der Substanz gemäß Schritt a1) mit der Substanz aus Schritt b1) zu der Substanz gemäß Formel C1) 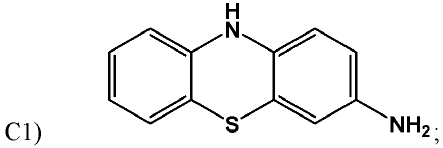
d1) Bereitstellung von Methylisobutylketon (MIBK) und Wasserstoff (H₂);
e1) Umsetzung der Substanz gemäß Formel C1) mit den Substanzen gemäß Schritt d1) zu der Substanz gemäß Formel I)

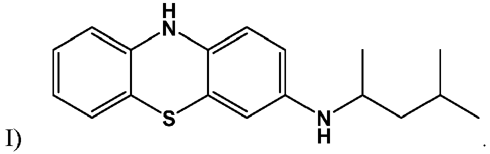

9. Verfahren zur Herstellung der Verbindung gemäß Formel I) nach Anspruch 1 umfassend wenigstens die folgenden Verfahrensschritte:
a2) Bereitstellung der Substanz gemäß Formel A2): 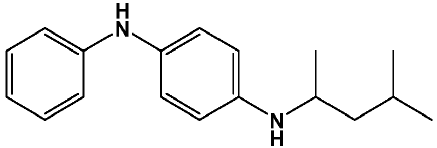 A2)
b2) Bereitstellung von elementarem Schwefel und o-Dichlorbenzol (ortho-Dichlorbenzol);
c2) Umsetzung der Substanz gemäß Schritt a2) mit den Substanzen gemäß Schritt b2) zu der Substanz gemäß Formel I)

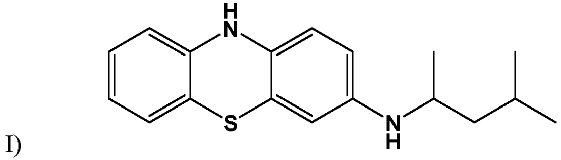

10. Verwendung der Verbindung gemäß Formel I) nach Anspruch 1 als Alterungsschutzmittel und/oder Ozonschutzmittel, insbesondere in Fahrzeugreifen und/oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

11. Verwendung der Verbindung gemäß Formel I) nach Anspruch 1 in Ölen, Schmierstoffen, wie insbesondere Treib- oder Betriebsstoffen für Motoren.

12. Verwendung der Verbindung gemäß Formel I) nach Anspruch 1 als Farbstoff in Fasern und/oder Polymeren und/oder Papier und/oder in (Streich-) Farben und Lacken.

## Claims

1. Compound of formula I):

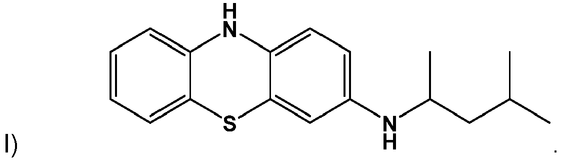

2. Rubber mixture containing the compound of formula I) according to Claim 1.

3. Rubber mixture according to Claim 2, **characterized in that** it contains at least one diene rubber.

4. Rubber mixture according to Claim 3, **characterized in that** it contains at least one diene rubber selected from the group consisting of natural polyisoprene (NR), synthetic polyisoprene (IR), butadiene rubber (BR), solution-polymerized styrene-butadiene rubber (SSBR), emulsion-polymerized styrene-butadiene rubber (ESBR), butyl rubber (IIR), and halobutyl rubber.

5. Vehicle tire comprising the rubber mixture according to any of Claims 2 to 4 in at least one component.

6. Vehicle tire according to Claim 5, **characterized in that** it contains at least one rubber mixture according to any of Claims 2 to 4 in at least one external component, wherein the external component is preferably a tread, a sidewall and/or a flange profile.

7. Process for producing the compound of formula I) according to Claim 1 comprising at least the following process steps:
a) providing the substance of formula A) 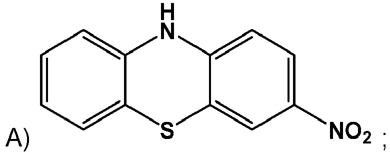
b) providing methyl isobutyl ketone (MIBK) and hydrogen (H₂);
c) reacting the substance according to step a) with the substances according to step b) to afford the substance of formula I)

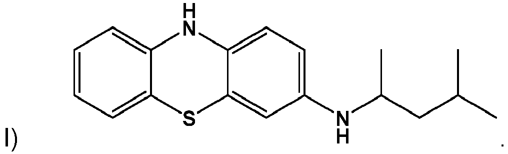

8. Process for producing the compound of formula I) according to Claim 1 comprising at least the following process steps:
a1) providing the substance of formula A) 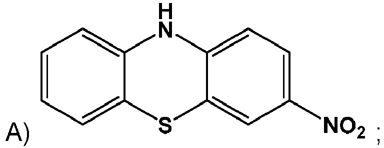
b1) providing a reducing agent, in particular tin(II) chloride dihydrate;
c1) reacting the substance according to step a1) with the substance from step b1) to afford the substance of formula C1) 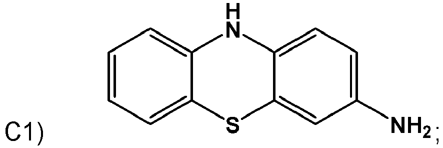
d1) providing methyl isobutyl ketone (MIBK) and hydrogen (H₂);
e1) reacting the substance of formula C1) with the substances according to step d1) to afford the substance of formula I)

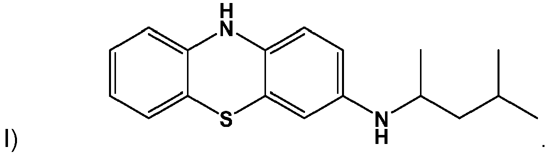

9. Process for producing the compound of formula I) according to Claim 1 comprising at least the following process steps:
a2) providing the substance of formula A2): 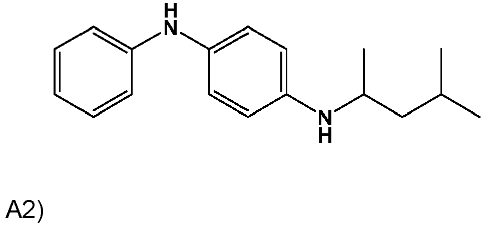
b2) providing elemental sulfur and o-dichlorobenzene (orthodichlorobenzene);
c2) reacting the substance according to step a2) with the substances according to step b2) to afford the substance of formula I)

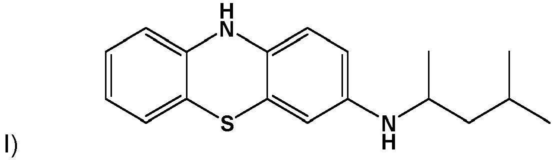

10. Use of the compound of formula I) according to Claim 1 as an aging stabilizer and/or antiozonant, in particular in vehicle tires and/or technical rubber articles, such as in particular an air spring, bellows, conveyor belt, belt, drive belt, hose, rubber band, profile, a seal, a membrane, tactile sensors for medical applications or robotics applications, or a shoe sole or parts thereof, and/or oils and/or lubricants.

11. Use of the compound of formula I) according to Claim 1 in oils and lubricants, such as in particular fuels or fluids for engines.

12. Use of the compound of formula I) according to Claim 1 as a dye in fibers and/or polymers and/or paper and/or in paints and coatings.

## Revendications

1. Composé selon la formule I) :

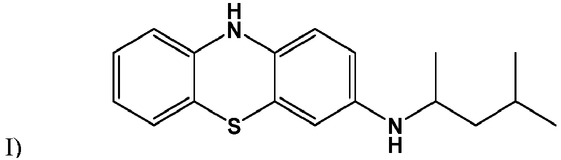

2. Mélange de caoutchouc contenant le composé selon la formule I) selon la revendication 1.

3. Mélange de caoutchouc selon la revendication 2, **caractérisé en ce qu'**il contient au moins un caoutchouc diénique.

4. Mélange de caoutchouc selon la revendication 3, **caractérisé en ce qu'**il contient au moins un caoutchouc diénique qui est choisi dans le groupe constitué par le polyisoprène naturel (NR), le polyisoprène synthétique (IR), le caoutchouc de butadiène (BR), le caoutchouc de styrène-butadiène polymérisé en solution (SSBR), le caoutchouc de styrène-butadiène polymérisé en émulsion (ESBR), le caoutchouc de butyle (UR) et le caoutchouc d'halogénobutyle.

5. Pneumatique de véhicule, qui présente le mélange de caoutchouc selon l'une des revendications 2 à 4 dans au moins un composant.

6. Pneumatique de véhicule selon la revendication 5, **caractérisé en ce qu'**il présente au moins un mélange de caoutchouc selon l'une des revendications 2 à 4 dans au moins un composant externe, le composant externe étant de préférence une bande de roulement, un flanc latéral et/ou un profilé de talon.

7. Procédé pour la préparation du composé selon la formule I) selon la revendication 1, comprenant au moins les étapes de procédé suivantes :
a) mise à disposition de la substance selon la formule A) 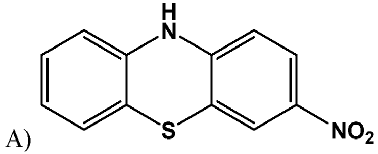
b) mise à disposition de méthylisobutylcétone (MIBK) et d'hydrogène (H₂) ;
c) transformation de la substance selon l'étape a) avec les substances selon l'étape b) en substance selon la formule I)

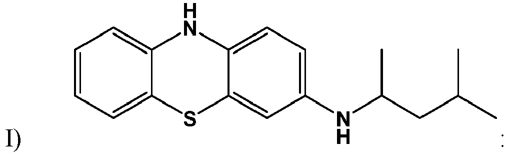

8. Procédé pour la préparation du composé selon la formule I) selon la revendication 1, comprenant au moins les étapes de procédé suivantes :
a1) mise à disposition de la substance selon la formule A) 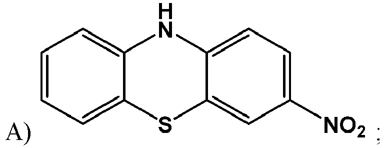
b1) mise à disposition d'un agent de réduction, en particulier du chlorure d'étain (II) dihydraté ;
c1) transformation de la substance selon l'étape a1) avec la substance de l'étape b1) en substance selon la formule C1) 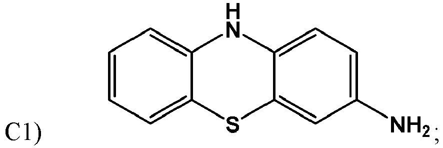
d1) mise à disposition de méthylisobutylcétone (MIBK) et d'hydrogène (H2) ;
e1) transformation de la substance selon la formule C1) avec les substances selon l'étape d1) en substance selon la formule I)

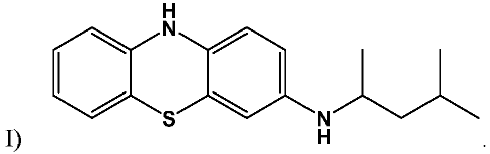

9. Procédé pour la préparation du composé selon la formule I) selon la revendication 1, comprenant au moins les étapes de procédé suivantes :
a2) mise à disposition de la substance selon la formule A2) : 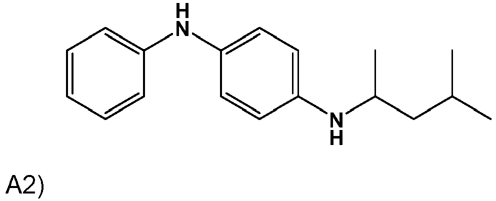
b2) mise à disposition de soufre élémentaire et d'o-dichlorobenzène (ortho-dichlorobenzène) ;
c2) transformation de la substance selon l'étape a2) avec les substances selon l'étape b2) en substance selon la formule I)

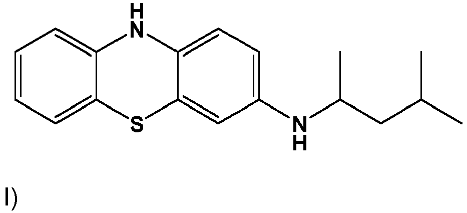

10. Utilisation du composé selon la formule I) selon la revendication 1 comme agent de protection contre le vieillissement et/ou agent de protection contre l'ozone, en particulier dans des pneumatiques de véhicule et/ou des articles techniques en caoutchouc, tels qu'en particulier d'un ressort à air, d'un soufflet, d'une bande transporteuse, d'une sangle, d'une courroie, d'un tuyau flexible, d'une bande en caoutchouc, d'un profilé, d'un joint d'étanchéité, d'une membrane, de capteurs tactiles destinés à des utilisations médicales ou des applications robotiques ou d'une semelle de chaussure ou de parties de ceux-ci et/ou comme huiles et/ou comme lubrifiants.

11. Utilisation du composé selon la formule I) selon la revendication 1 dans des huiles, des lubrifiants, tels qu'en particulier des carburants ou des consommables pour des moteurs.

12. Utilisation du composé selon la formule I) selon la revendication 1 comme colorant dans des fibres et/ou des polymères et/ou du papier et/ou dans des peintures (d'enduction) et des vernis.
